Europäisches Patentamt

European Patent Office

Office européen des brevets

(19)

(11) Veröffentlichungsnummer: **0 153 908**
**A1**

(12) # EUROPÄISCHE PATENTANMELDUNG

(21) Anmeldenummer: **85810075.3**

(22) Anmeldetag: **25.02.85**

(51) Int. Cl.⁴: **C 07 D 213/807**

(30) Priorität: **01.03.84 CH 1011/84**

(43) Veröffentlichungstag der Anmeldung:
**04.09.85 Patentblatt 85/36**

(84) Benannte Vertragsstaaten:
**CH DE FR GB LI NL**

(71) Anmelder: **CIBA-GEIGY AG**
**Postfach**
**CH-4002 Basel(CH)**

(72) Erfinder: **Weis, Claus D., Dr.**
**Hangelimattweg 112**
**CH-4148 Pfeffingen(CH)**

(54) Verfahren zur Herstellung von Pyridin-2,3-dicarbonsäure.

(57) Verfahren zur Herstellung von Pyridin-2,3-dicarbonsäure, indem man 5-Halogen-8-hydroxy-chinolin oder dessen Salze, wobei Halogen Brom, Fluor, Jod oder vorzugsweise Chlor bedeutet, mit Salpetersäure in Gegenwart einer Vanadiumverbindung und vorzugsweise Salzsäure bei einer Temperatur von 70 bis 110°C katalytisch oxydiert.

Croydon Printing Company Ltd.

EP 0 153 908 A1

CIBA-GEIGY AG 1-14781/+

Basel (Schweiz)

Verfahren zur Herstellung von Pyridin-2,3-dicarbonsäure

Die vorliegende Erfindung betrifft ein eigenartiges Verfahren zur
Herstellung von Pyridin-2,3-dicarbonsäure durch katalytische Oxidation
einer 5-Halogen-8-hydroxy-chinolinverbindung und insbesondere von
5-Chlor-8-hydroxychinolin.

Es ist bekannt, Pyridin-2,3-dicarbonsäure durch Oxidation von 8-Hydroxychinolin mit Salpetersäure bei 0 bis 5°C herzustellen (vgl. Houben-
Weyl, Methoden der organischen Chemie, 4/1a (1955) Seiten 731-732).
Gemäss der DE-B-455 386 kann Pyridin-2,3-dicarbonsäure auch durch
Einwirkung von Salpetersäure auf 6-Chlor-8-hydroxychinolin bei einer
höheren Temperatur von 70 bis 90°C erhalten werden.

Ebenso ist aus der genannten Houben-Weyl-Literaturstelle bekannt, dass
Chinolin mit Salpetersäure/Schwefelsäure und Ammoniumvanadat als
Katalysator zu Nicotinsäure abgebaut werden kann.

Es wurde nun gefunden, dass Pyridin-2,3-dicarbonsäure in guter
Ausbeute und mit hoher Qualität hergestellt werden kann, wenn
5-Halogen-8-hydroxy-chinolin mit Salpetersäure unter Mitverwendung
von spezifischen Katalysatoren oxidiert wird.

Gegenstand vorliegender Erfindung ist daher ein Verfahren zur
Herstellung von Pyridin-2,3-dicarbonsäure, welches dadurch gekennzeichnet ist, dass man 5-Halogen-8-hydroxychinolin oder dessen Salz,
wobei Halogen Brom, Fluor, Jod oder vorzugsweise Chlor bedeutet, mit
Salpetersäure in Gegenwart einer Vanadiumverbindung bei einer Temperatur von 70 bis 110°C katalytisch oxidiert.

- 2 -

Das grundsätzliche Merkmal der vorliegenden Erfindung besteht darin, dass eine 8-Hydroxychinolin-Verbindung, die ein Halogenatom, insbesondere ein Chloratom in 5-Stellung enthält, mit Salpetersäure oxidiert wird. Das charakteristische Merkmal der vorliegenden Erfindung besteht darin, dass man die Oxidation unter den genannten Bedingungen in Gegenwart eines Katalysators vornimmt, der aus Vanadiumverbindungen ausgewählt ist. Vorzugsweise erfolgt die Oxidation in Gegenwart von Salzsäure.

Bei den als Katalysator in Betracht kommenden Vanadiumverbindungen handelt es sich vorteilhafterweise um solche, die in Salpetersäure oder im Gemisch aus Salpetersäure und Salzsäure löslich sind. Besonders geeignete Katalysatoren sind Vanadiumsulfat, Alkalimetallmetavanadate, wie z.B. deren Kalium- oder Natriumsalze oder vor allem Ammoniumvanadat.

Die erfindungsgemäss verwendbaren Katalysatoren zeigen einen ausgezeichneten katalytischen Effekt und können in geringen Mengen eingesetzt werden. Ferner können die Katalysatoren nach der Oxidation durch Abfiltrieren der erhaltenen Pyridin-2,3-dicarbonsäure und Waschen mit Wasser leicht abgetrennt werden, so dass man die Pyridin-2,3-dicarbonsäure mit hoher Reinheit erhält. Ausserdem kann die filtrierte und zur Trockene eingedampfte Katalysatorlösung zu einem kontinuierlichen Oxidationsprozess unter beträchtlicher Verbesserung der Ausbeute wiedereingesetzt werden.

Die Katalysatormenge liegt vorteilhafterweise in einem Bereich von 0,001 bis 10 Gew.-% oder 0,01 bis 10 Gew.-%, vorzugsweise 0,1 bis 5 Gew.-%, bezogen auf die eingesetzte Menge des Ausgangsmaterials. Bevorzugt wird Ammoniumvanadat in einer Menge von 1 bis 3,5 Gew.-%, bezogen auf die Menge des Ausgangsstoffes, verwendet.

Die als Ausgangsstoffe in Frage kommenden 5-Halogen-8-hydroxy-chino- line könnnen als freie Basen oder in Form von Salzen, vorzugsweise Säuresalzen, z.B. Hydrogensulfaten oder Hydrohalogeniden wie Hydrobromiden oder vorzugsweise Hydrochloriden vorliegen. Als salzbildende Säuren kommen organische oder vor allem anorganische Säuren in Betracht, z.B. Ameisensäure, Essigsäure, Schwefelsäure, Bromwasserstoffsäure oder in erster Linie Salzsäure.

Als Ausgangsstoffe sind beispielsweise geeignet:
5-Brom-8-hydroxy-chinolin, 5-Fluor-8-hydroxy-chinolin, 5-Jod-8-hydroxy-chinolin und vor allem 5-Chlor-8-hydroxy-chinolin sowie die entsprechenden Chinolinium-Hydrogensulfate, Chinolinium-Hydrochloride oder Chinolinium-Hydrobromide.

Isomere 6-Halogen-8-hydroxy-chinoline bzw. deren Salze hingegen ergeben unter den erfindungsgemässen Bedingungen keine Pyridin-2,3-dicarbonsäure.

Beim erfindungsgemässen Verfahren wird Salpetersäure als Oxidationsmittel verwendet. Die Salpetersäure kann in Form von wässrigen Lösungen mit Konzentrationen im Bereich von 40 bis 90 Gew.-%, vorzugsweise 50 bis 70 Gew.-% oder als Gemisch von 64 - 68 %iger Salpetersäure mit 100 %iger Salpetersäure eingesetzt werden. Die effektive Menge des Oxidationsmittels liegt vorteilhafterweise bei einem Molequivalent von 1,1 bis 4, vorzugsweise 1,2 bis 1,9 bezogen auf die 5-Halogen-8-hydroxy-chinolinverbindung bzw. deren Salz.

Vorzugsweise wird die Salpetersäure im Gemisch mit konzentrierter Salzsäure (35-40 %) eingesetzt, wobei das Mischungsverhältnis von $HNO_3$ und HCl zweckmässigerweise bei 5:1 bis 1:1, vorzugsweise 3:1 bis 2:1 liegt.

Da die Reaktion exotherm verläuft, ist es im allgemeinen zweckmässig im Temperaturbereich von 70 bis 110°C, bevorzugt von 80 bis 100°C und insbesondere zwischen 90 und 95°C zu arbeiten. Ueberschreiten der Obergrenze des Temperaturbereiches von 110°C ist möglich, bringt jedoch den Nachteil eines grösseren Aufwandes für die Kühlung und die Gefahr mit sich, dass Decarboxylierung zu Nicotinsäure eintritt.

Die nach dem erfindungsgemässen Verfahren erhältliche Pyridin-2,3-dicarbonsäure stellt ein wertvolles Ausgansprodukt für die verschiedenartigsten chemischen Synthesen dar. Insbesondere ist sie als Ausgangsstoff für die Herstellung von 1-Azaanthrachinonfarbstoffen oder von 3,3-disubstituierten Azaphthaliden oder basisch substituierten Azafluoranen, die als Farbbildner für druck- oder wärmeempfindliche Aufzeichnungsmaterialien eingesetzt werden, geeignet.

Weitere Einzelheiten sind aus den nachfolgenden, den Erfindungsgegenstand in keiner Weise beschränkenden Beispielen ersichtlich. Dabei bedeuten Prozente Gewichtsprozente.

Beispiel 1: 1,0 g Ammoniumvanadat werden unter Rühren in 300 ml konzentrierter Salpetersäure (64%) gelöst. Hierauf fügt man 30 g gepulvertes 5-Chlor-8-hydroxychinolin hinzu, worauf die Temperatur auf 40-45°C ansteigt. Nach etwa einer Minute beginnt die Reaktion unter Entwicklung von nitrosen Gasen und die Temperatur steigt langsam auf 91-93°C an. Man lässt das Reaktionsgemisch auf 25°C abkühlen und dampft die Lösung im Rotationsverdampfer zur Trockene ein. Anschliessend lässt man zweimal je 150 ml Wasser zufliessen und dampft wieder zur Trockene ein. Zum Rückstand gibt man 50 ml Eiswasser zu, worauf die erhaltenen Kristalle abfiltriert und mit 50 ml Eiswasser gewaschen werden. Nach dem Trocknen der Kristalle über Phosphorpentoxid in Vakuum erhält man 22,3 g der Pyridin-2,3-dicarbonsäure. Die Ausbeute beträgt 79,7 % der Theorie.

Analyse der Pyridin-2,3-dicarbonsäure ($C_7H_5NO_4$)

| | | | |
|---|---|---|---|
| ber.: | C 50,31 % | H 3,02 % | N 8,38 % |
| gef.: | C 49,9 % | H 3,1 % | N 8,4 % |

Beispiel 2: Die gemäss Beispiel 1 durch Filtrieren erhaltene Katalysatorlösung wird ebenfalls zur Trockene eingedampft, worauf der Rückstand in 300 ml konzentrierter Salpetersäure gelöst wird. Zu dieser Lösung gibt man 30 g 5-Chlor-8-hydroxychinolin. Die Temperatur steigt im Verlaufe von 5-10 Minuten auf 95°C an. Man lässt dann auf 25°C abkühlen und arbeitet die Lösung auf so wie in Beispiel 1 beschrieben. Man erhält nach dem Trocknen des Produktes 27,5 g Pyridin-2,3-dicarbonsäure mit einem Schmelzpunkt von 183-186°C.
Die Ausbeute beträgt 98 % der Theorie.

Die abfiltrierte Katalysatorlösung kann nach nochmaligem Eindampfen wieder verwendet werden.

Beispiel 3: In 5 ml konzentrierter Schwefelsäure suspendiert man 1 g Vanadiumpentoxid und erwärmt die Suspension unter Rühren auf 155°C, worauf das Vanadiumpentoxid unter Bildung von Vanadiumsulfat in Lösung geht. Man gibt diese Lösung zu 300 ml konzentrierter Salpetersäure (64%), wobei eine klare gelbe Lösung erhalten wird.

Zu dieser neuen Lösung gibt man bei 20-25°C 30 g 5-Chlor-8-hydroxychinolin und verfährt wie in Beispiel 1 beschrieben. Man erhält 20 g der Pyridin-2,3-dicarbonsäure. Die Ausbeute beträgt 75,5 % der Theorie.

Beispiel 4 : Zu einer Lösung von 1 g Ammoniumvanadat in 300 ml 64%-iger Salpetersäure werden 30 g 5-Brom-8-hydroxychinolin zugegeben, worauf die Temperatur auf 45-50°C ansteigt. Hierauf beginnt die Reaktion unter Entwicklung von nitrosen Gasen und die Temperatur steigt auf 93°C an. Man lässt das Reaktionsgemisch auf Raumtemperatur abkühlen und arbeitet die erhaltene Lösung auf wie in Beispiel 1 beschrieben. Man erhält 22 g der Pyridin-2,3-dicarbonsäure. Die Ausbeute beträgt 79% der Theorie.

Beispiel 5: In eine Lösung von 1,5 g Ammoniumvanadat in 300 ml
64 %iger Salpetersäure werden 54 g 5-Chlor-8-hydroxychinolinium-
Hydrogensulfat eingetragen, worauf die Temperatur unter Entwicklung
von nitrosen Gasen auf 88°C ansteigt. Nach Beendigung der Reaktion
lässt man abkühlen und dampft die Lösung am Rotationsverdampfer
ein. Der Rückstand wird mit einer Lösung von 35 g Natriumbicarbonat in 200 ml Wasser behandelt. Man erhält einen beigefarbenen
Niederschlag, der abfiltriert und mit 50 ml Eiswasser gewaschen
wird. Die Ausbeute an Pyridin-2,3-dicarbonsäure beträgt 17 g.

Beispiel 6: In eine Lösung von 1 g Ammoniumvanadat in 300 ml 64%-
iger Salpetersäure werden 30 g 5-Chlor-8-hydroxy-chinolinium-Hydrochlorid eingetragen, wobei die Temperatur auf 45°C ansteigt. Hierauf
steigt die Temperatur weiter auf 93°C unter Entwicklung von nitrosen
Gasen. Man lässt dann abkühlen und arbeitet die Lösung auf wie in
Beispiel 1 beschrieben. Die Ausbeute an Pyridin-2,3-dicarbonsäure
beträgt 22 g.

Das während der Aufarbeitung erhaltene Filtrat, das den Katalysator
enthält, wird zur Trockene eingedampft und in einem weiteren
Ansatz zur Herstellung der Pyridin-2,3-dicarbonsäure wieder
verwendet.

Beispiel 7: In eine Mischung von 100 ml 100 %iger Salpetersäure
und 200 ml 64 %iger Salpetersäure werden 0,1 g Ammoniumvanadat gelöst.
Zu dieser auf 5°C abgekühlten Lösung fügt man unter Rühren 30 g
5-Chlor-8-hydroxychinolin langsam bei Aussenkühlung so hinzu, dass
die Temperatur nicht über 25°C ansteigt. Alsdann entfernt man das
Kühlbad, worauf die Temperatur auf 50 - 55°C ansteigt. Es erfolgt eine
starke Entwicklung von nitrosen Gasen und die Temperatur steigt weiter
auf 87°C an.

- 7 -

Man lässt abkühlen und dampft die Lösung bei 70°C/39 mbar zur
Trockene ein. Die zurückbleibenden Kristalle werden mit 50 ml Eiswasser versetzt und abfiltriert. Man erhält nach dem Trocknen 25,2 g
2,3-Pyridindicarbonsäure mit einem Schmelzpunkt von 182°C (Zersetzung).

Beispiel 8: In eine Lösung von 20 mg Ammoniumvanadat in 90 ml 64 %iger
Salpetersäure und 30 ml konzentrierter Salzsäure (38 %) werden 30 g
5-Chlor-8-hydroxychinolin bei 22 - 25°C im Verlauf von 15 Minuten eingetragen, worauf die Temperatur im Verlaufe von 20 Minuten auf 43°C
ansteigt. Man erwärmt auf 65°C, worauf eine Gasentwicklung einsetzt
und Kristalle aus der Lösung ausfallen. Die Temperatur steigt auf
75 - 80°C an. Wenn die Gasentwicklung aufgelöst hat, wird die Temperatur durch Erwärmen für 1 1/2 Stunden auf 70 - 75°C gehalten. Danach werden die Säuren durch Verdampfen bei 70°C/39 mbar entfernt.
Der kristalline Rückstand wird mit 50 ml Eiswasser verrührt und abfiltriert. Nach dem Trocknen erhält man 27,6 g Pyridin-2,3-dicarbonsäure mit einem Schmelzpunkt von 181 - 182°C. (Zersetzung).

Wird bei Einsetzen der Reaktion keine Aussenkühlung vorgenommen, so
steigt die Temperatur auf 85°C an. Man erhält dann bei analoger
Aufarbeitung 28,5 g Pyridin-2,3-dicarbonsäure mit einem Schmelzpunkt
von 182°C (Zersetzung).

Beispiel 9: Zu einer Lösung von 90 ml 64 %iger Salpetersäure und
30 ml konzentrierter Salzsäure (38 %) gibt man 20 mg Ammoniumvanadat.
Hierauf fügt man unter Rühren bei Zimmertemperatur 44 g 5-Chlor-
8-hydroxychinolinhydrogensulfat hinzu. Man erwärmt langsam auf 60°C
und erhält eine klare Lösung, wobei die Reaktion unter Gasentwicklung
einsetzt und die Temperatur auf 84°C ansteigt. Nach dem Abfallen der
Temperatur wird durch Heizen die Temperatur für eine Stunde bei 72 -
73°C gehalten. Die erhaltene Suspension wird am Verdampfer eingeengt
und zum halbfesten Rückstand eine Lösung von 5 g Natriumhydroxid
in 60 ml Wasser gegeben. Die Suspension wird in Eiswasser gekühlt,

- 8 -

worauf die erhaltenen Kristalle abfiltriert und getrocknet werden.
Man erhält 17,3 g Pyridin-2,3-dicarbonsäure. Smp. 182°C (Zersetzung).

Beispiel 10: Zu einer Mischung von 90 ml 64 %iger Salpetersäure und
30 ml konzentrierter Salzsäure (38 %) werden 20 mg Natriumvanadat
zugegeben. Hierauf trägt man unter Rühren und Kühlung 30 g 5-Chlor-
8-hydroxychinolin in die Lösung ein. Man rührt 30 Minuten bei
ansteigender Temperatur (25-40°C) und erwärmt auf 65°C. Die Reaktion
setzt unter Gasentwicklung und Temperatursteigerung bis zu 87°C
ein. Man hält das Reaktionsgemisch 15 Minuten bei 85 - 87°C und isoliert das Produkt wie in Beispiel 8 beschrieben. Man erhält 27 g
Pyridin-2,3-dicarbonsäure in Form gelblicher Kirstalle. Smp.
181 - 182°C.

Patentansprüche

1. Verfahren zur Herstellung von Pyridin-2,3-dicarbonsäure, dadurch gekennzeichnet, dass man 5-Halogen-8-hydroxychinolin oder dessen Salz, worin Halogen Brom, Chlor, Fluor oder Jod bedeutet, mit Salpetersäure in Gegenwart einer Vanadiumverbindung bei einer Temperatur von 70 bis 110°C katalytisch oxidiert.

2. Verfahren gemäss Anspruch 1, dadurch gekennzeichnet, dass man die Oxidation zudem in Gegenwart von Salzsäure durchführt, wobei das Verhältnis von $HNO_3$ zu HCl 5:1 bis 1:1 beträgt.

3. Verfahren gemäss einem der Ansprüche 1 und 2, dadurch gekennzeichnet, dass die Vanadiumverbindung in einer Menge von 0,001 bis 10 Gew.-% bezogen auf die Menge des 5-Halogen-8-hydroxychinolins oder dessen Salz verwendet wird.

4. Verfahren gemäss einem der Ansprüche 1 bis 3, dadurch gekennzeichnet, dass man 5-Chlor-8-hydroxychinolin zu Pyridin-2,3-dicarbonsäure oxidiert.

5. Verfahren gemäss einem der Ansprüche 1 bis 4, dadurch gekennzeichnet, dass man die Oxidation in Gegenwart von Ammoniumvanadat, Alkalimetavanadat oder Vanadiumsulfat durchführt.

6. Verfahren gemäss einem der Ansprüche 1 bis 5, dadurch gekennzeichnet, dass man die Oxidation bei einer Temperatur von 90 bis 95°C durchführt.

7. Die nach dem Verfahren gemäss einem der Ansprüche 1 bis 6 hergestellte Pyridin-2,3-dicarbonsäure.

FO 7.1/PE/rz*

| | EINSCHLÄGIGE DOKUMENTE | | EP 85810075.3 | |
|---|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) | |
| A | <u>DE - A1 - 3 150 005</u> (RÜTGERSWERKE)<br><br>* Anspruch *<br><br>-- | 1,4,5 | C 07 D 213/807 | |
| A | <u>DE - C - 945 147</u> (RIEDEL)<br><br>* Anspruch *<br><br>-- | 1,5 | | |
| D,A | <u>DE - C - 455 386</u> (GESELLSCHAFT F. CHEMIE)<br><br>* Anspruch *<br><br>---- | 1,2 | | |
| | | | **RECHERCHIERTE SACHGEBIETE (Int. Cl.4)** | |
| | | | C 07 D 213/00 | |

Der vorliegende Recherchenbericht wurde für alle Patentansprüche erstellt.

| Recherchenort | Abschlußdatum der Recherche | Prüfer |
|---|---|---|
| WIEN | 31-05-1985 | HOCHHAUSER |